# EUROPEAN PATENT APPLICATION

(11) **EP 2 594 311 A2**
(43) Date of publication of application: **22.05.2013**
(21) Application number: 13155418.0
(22) Date of filing: 06.03.2009
(51) Int. Cl.: A61M 25/10, A61L 29/08, A61M 25/00, A61L 29/16, B29D 23/00

(54) **Balloon catheter devices with sheath covering**

(30) Priority: 06.03.2008 US 34328 P
(62) Divisional of application: 09716409.9
(71) Applicant: Boston Scientific Scimed, Inc., Maple Grove, MN 55311 (US)
(72) Inventor: The designation of the inventor has not yet been filed
(74) Representative: Vossius & Partner

(57) **Abstract**

The present application relates to a medical device comprising a catheter; a balloon mounted on the catheter; a sheath disposed around the balloon, wherein the sheath comprises nanofibers; and the therapeutic agent is retained on the medical device by the sheath. The agent may be contained between the sheath and the balloon or dispersed in the space between the nanofibres.

## Description

### RELATED APPLICATIONS

This application claims the benefit of provisional application Serial No. 61/034,328 (filed 6 March 2008), which is incorporated by reference herein.

### TECHNICAL FIELD

The present invention relates to medical devices, more particularly, to catheter devices.

### BACKGROUND

Catheters are used in a wide variety of minimally-invasive or percutaneous medical procedures. Balloon catheters having drug coatings may be used to treat diseased portions of blood vessels. Typically, the balloon is inserted through a peripheral blood vessel and then guided via a catheter through the vascular system to the target intravascular site. However, as the balloon travels through the vascular system, the flow of blood may wash away some of the drug coating. In addition, the control of the timing, location, and/or duration of the drug release can be an issue. Therefore, there is a need for improved catheter-based devices for drug delivery to an intravascular site.

### SUMMARY

In one embodiment, the present invention provides a medical device comprising: a catheter; a balloon mounted on the catheter; one or more sheaths disposed around the balloon, wherein the one or more sheaths are slidable over the balloon; and a therapeutic agent disposed between the surface of the balloon and the one or more sheaths. The therapeutic agent may be formulated to include a pharmaceutically-acceptable lubricant material.

In another embodiment, the present invention provides a medical device comprising: a catheter; a balloon mounted on the catheter; a sheath disposed around the balloon, wherein the sheath has a weakened portion, and wherein the sheath is disrupted at the weakened portion when the balloon is expanded; and a therapeutic agent disposed between the surface of the balloon and the sheath.

In another embodiment, the present invention provides a medical device comprising: a catheter; a balloon mounted on the catheter; a sheath disposed around the balloon, wherein the sheath comprises nanofibers; and a therapeutic agent retained on the medical device by the sheath.

In another embodiment, the present invention provides a medical device comprising: a catheter; a balloon mounted on the catheter; a sheath disposed around the balloon, wherein the sheath comprises a body and a plurality of reservoirs that are in communication with the external surface of the body of the sheath, wherein the reservoirs are longitudinally-extending channels within the body of the sheath; and a therapeutic agent contained in the reservoirs.

In another embodiment, the present invention provides a method for making a medical device, comprising: providing a balloon; disposing over the surface of the balloon, a layer of therapeutic agent; embossing a pattern onto the layer of therapeutic agent; and disposing a sheath over the layer of therapeutic agent.
1. A medical device comprising:
   a catheter;
   a balloon mounted on the catheter;
   a sheath disposed around the balloon, wherein the sheath has a weakened portion, and wherein the sheath is disrupted at the weakened portion when the balloon is expanded; and
   a therapeutic agent disposed between the surface of the balloon and the sheath.
2. The medical device of aspect 1, wherein the weakened portion is a perforation line.
3. The medical device of aspect 1, wherein the weakened portion is a plurality of perforation lines that are arranged in a grid-like pattern.
4. The medical device of aspect 3, wherein the disruption of the sheath along the perforation lines creates fragments.
5. A medical device comprising:
   a catheter;
   a balloon mounted on the catheter;
   a sheath disposed around the balloon, wherein the sheath comprises nanofibers; and
   a therapeutic agent retained on the medical device by the sheath.
6. The medical device of aspect 5, wherein the therapeutic agent is disposed between the surface of the balloon and the sheath.
7. The medical device of aspect 6, wherein the nanofibers are elastic, and wherein stretching of the nanofibers upon balloon expansion enlarges the spacing between the nanofibers.
8. The medical device of aspect 6, wherein the therapeutic agent is squeezed through spaces between the nanofibers when the balloon is inflated.
9. The medical device of aspect 5, wherein the nanofibers comprise a biodegradable polymer.
10. The medical device of aspect 5, wherein the therapeutic agent is dispersed in the space between the nanofibers.
11. A medical device comprising:
   a catheter;
   a balloon mounted on the catheter;
   a sheath disposed around the balloon, wherein the sheath comprises a body and a plurality of reservoirs that are in communication with the external surface of the body of the sheath, wherein the reservoirs are longitudinally-extending channels within the body of the sheath; and
   a therapeutic agent contained in the reservoirs.
12. The medical device of aspect 11, wherein expansion of the balloon applies pressure to the reservoirs and causes the release of the therapeutic agent.
13. The medical device of aspect 11, wherein the sheath has a non-linear compliance curve.
14. The medical device of aspect 11, wherein the sheath comprises an elastomeric mesh.
15. A medical device comprising:
   a catheter; and
   a balloon mounted on the catheter, wherein the wall of the balloon comprises:
      (a) a plurality of micro-sized reservoirs containing a therapeutic agent; and
      (b) an actuatable cap covering each of the reservoirs, wherein the caps open upon actuation.
16. The medical device of aspect15, wherein the caps are actuatable by the application of an electric current or temperature change.
17. The medical device of aspect 15, wherein the actuatable caps are formed of a shape memory material.
18. A medical device comprising:
   a catheter;
   a balloon mounted on the catheter;
   one or more sheaths disposed around the balloon, wherein the one or more sheaths are slidable over the balloon; and
   a therapeutic agent disposed between the surface of the balloon and the one or more sheaths, wherein the therapeutic agent is formulated to include a pharmaceutically-acceptable lubricant material.
19. The medical device of aspect 1, wherein the balloon has a proximal end and a distal end, wherein one sheath is positioned at the proximal end and another sheath is positioned at the distal end of the balloon, and wherein the proximal sheath retracts proximally and the distal sheath retracts distally upon expansion of the balloon.
20. A method for making a medical device, comprising:
   providing a balloon;
   disposing over the surface of the balloon, a layer of therapeutic agent;
   embossing a pattern onto the layer of therapeutic agent; and
   disposing a sheath over the layer of therapeutic agent.
21. The method of aspect20, wherein the step of disposing the sheath comprises depositing a brittle film onto the layer of therapeutic agent.
22. The method of aspect 21, wherein the brittle film has thin portions that substantially follow the pattern on the layer of therapeutic agent.

### BRIEF DESCRIPTION OF THE DRAWINGS

FIG. 1 shows a cross-section side view of a portion of a sheath according to an embodiment. It is to be noted that certain features in these drawings have been exaggerated to more clearly show details thereof. For example, in FIG. 1, the size of the fibers and therapeutic agents are exaggerated relative to the thickness of the sheath.

FIG. 2 shows a magnified side view of a surface of a sheath according to another embodiment.

FIG. 3 shows a magnified side view of a surface of a sheath according to another embodiment.

FIGS. 4A and 4B show a catheter device according to another embodiment. FIG. 4A shows the catheter device with the balloon in a deflated stated. FIG. 4B shows the catheter device with the balloon inflated.

FIGS. 5A-5C show a catheter device according to another embodiment. FIG. 5A shows the catheter device with the balloon in a deflated state. FIG. 5B shows a transverse cross-section view of the balloon taken at arrow A in FIG. 5A. FIG. 5C shows a transverse cross-section view of the balloon taken at arrow A in FIG. 5A with the balloon inflated.

FIGS. 6A-6D show a catheter device according to another embodiment. FIGS. 6A and 6B (transverse cross-section view) show the catheter device with the balloon in a deflated state. FIGS. 6C and 6D (transverse cross-section view) show the catheter device with the balloon inflated.

FIGS. 7A-7D show a catheter device according to another embodiment. FIGS. 7A and 7B (enlarged view of the elastomeric mesh) show the catheter device with the balloon in a deflated state. FIGS. 7C and 7D (enlarged view of the elastomeric mesh) show the catheter device with the balloon inflated.

FIGS. 8A and 8B show side views of a catheter device according to another embodiment.

FIGS. 9A and 9B show side views of a catheter device according to another embodiment.

FIGS. 10A and 10B show side views of a catheter device according to another embodiment.

FIGS. 11A and 11B show side views of a catheter device according to another embodiment.

FIGS. 12A-12C show transverse cross-sections of a portion of a balloon according to an embodiment.

FIGS. 13A-13C show images of a nanofiber sheath wrapped around a balloon in its folded configuration.

FIGS. 14A and 14B show images of a nanofiber sheath wrapped around a balloon in its inflated configuration.

FIGS. 15A-15C show the wall of a balloon according to yet another embodiment. FIG. 15A shows a cross-section perspective view of a fragment of the balloon wall. FIG. 15B shows a cross-sectional side view of the balloon wall shown in FIG. 15A. FIG. 15C shows the balloon wall of FIG. 15B after the caps have been actuated to open.

It is to be noted that certain features in these drawings have been exaggerated to more clearly show details thereof. For example, the diameter of an uninflated balloon relative to the thickness of the catheter shaft may be smaller than what is depicted in the drawings.

### DETAILED DESCRIPTION

Catheter devices of the present invention use an expandable balloon for delivering a therapeutic agent to a target site in the body. The balloon is designed to be insertable in the body via a catheter. The therapeutic agent can be associated with the balloon in any of various ways, as further described below. Any of various mechanisms conventionally used for the delivery, actuation, or expansion (e.g., by inflation) of balloon catheter devices may be used in the present invention. The balloon catheter may be designed similar to those that have been known in the art, including but not limited to angioplasty catheters, stent delivery catheters, inflation catheters, and/or perfusion catheters. The catheter devices of the present invention may be used in conjunction with other drug delivery devices, such as stents.

In one aspect of the present invention, one or more sheaths are disposed around the balloon. The sheath may be made of various types of materials, and in some cases, the sheath may be formed of a permeable or semi-permeable material to allow the passage of the therapeutic agent through the sheath. For example, the sheath may be made of expanded polytetrafluoro-ethylene (ePTFE), which can be made by expanding PTFE tubing under controlled conditions during the manufacturing process. This expansion process alters the physical properties of the PTFE tubing such that microscopic pores are created in the tube. The sheath can also be made from various other types of polymeric materials, such as polyolefin copolymers, fluoropolymers, noncompliant polyethylene terephthalate (PET), polyimide, nylon, polyethylene, PEBAX, or the like. The sheath may have varying degrees of compliance depending upon the type of balloon being used and the particular application. The sheath may be attached to the surface of the balloon (e.g., at one or more points or continuously) or the sheath may be free of any attachments to the surface of the balloon (e.g., the sheath is "free-floating" over the balloon surface).

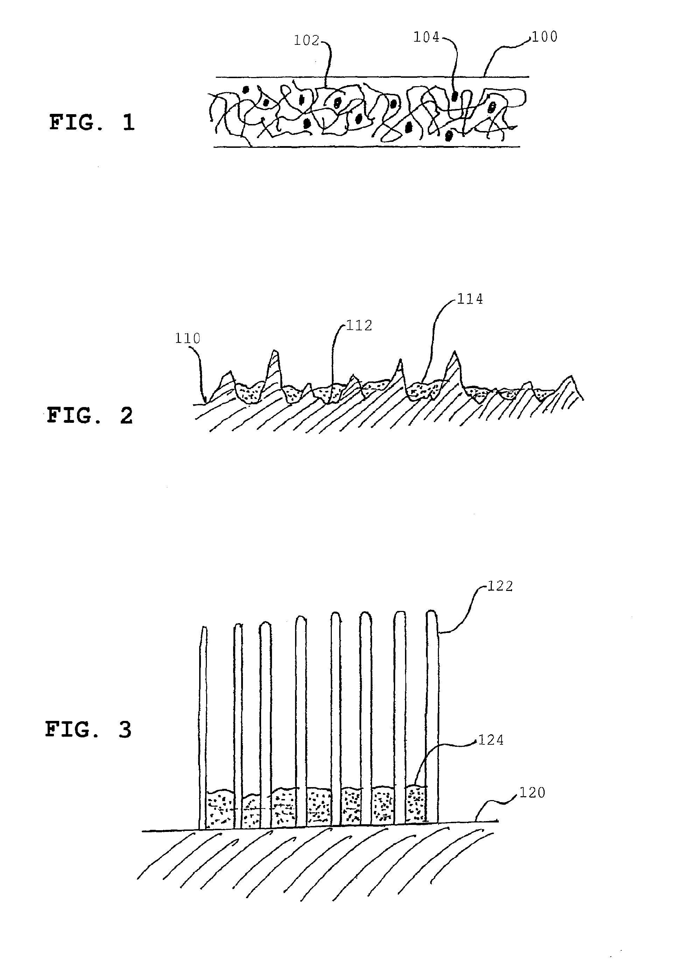

Further, a therapeutic agent can be disposed over the sheath, within the sheath, or between the balloon and the sheath. The therapeutic agent can be associated with the sheath in various ways. For example, referring to FIG. 1, a sheath 100 has a fibrous structure (e.g., a fibrous mat-like structure) containing a network of fibers 102, and therapeutic agent 104 is dispersed within the network of fibers 102. In another example, referring to the magnified side view shown in FIG. 2, the surface 110 of a sheath is rough-textured and therapeutic agent 114 is dispersed in the interstices 112 of the rough-textured surface. In another example, referring to the magnified side view shown in FIG. 3, a surface 120 of a sheath has numerous pili 122 (e.g., hair-like projections) extending from surface 120, and therapeutic agent 124 is dispersed between pili 122.

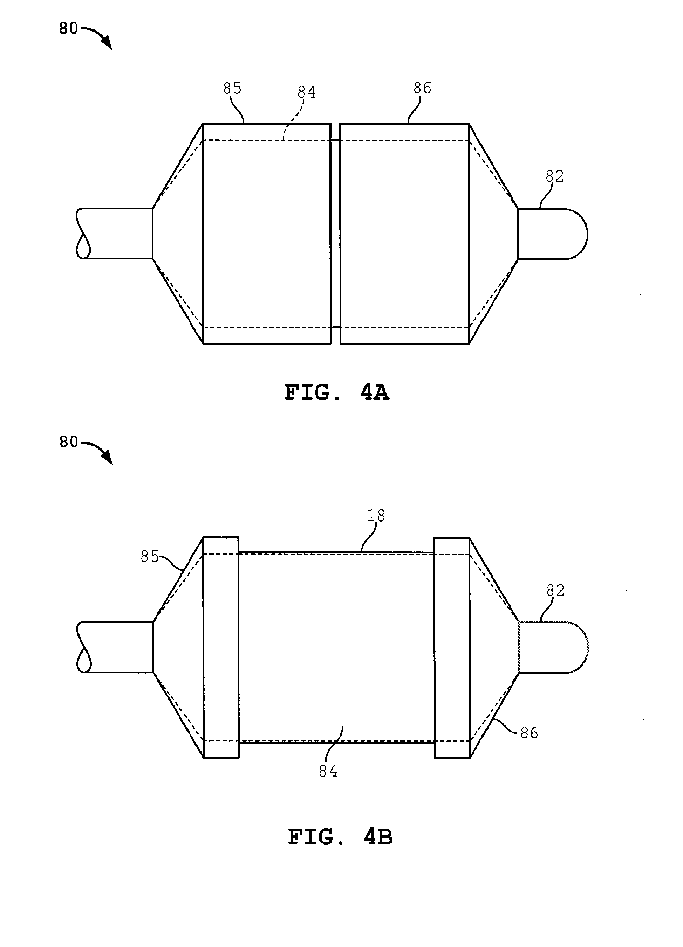

In certain embodiments, the sheath is designed to retract away from the balloon as the balloon is expanded. For example, referring to the embodiment shown in FIGS. 4A and 4B, a catheter device 80 comprises a balloon 84 mounted on an elongate shaft 82 (note that balloon 84 may have a smaller unexpanded profile relative to the shaft 82 than that illustrated in FIG. 4A). Balloon 84 is coated with a therapeutic agent 18 which is formulated to include a pharmaceutically-acceptable lubricant material. The pharmaceutically-acceptable lubricant material can either be incorporated into a matrix comprising the therapeutic agent or the therapeutic agent can be directly disposed on the outer surface of balloon 84 and the lubricant can be placed over the therapeutic agent as a top coat. The proximal half of balloon 84 is covered by a proximally located sheath 85 and the distal half of balloon 84 is covered by a distally located sheath 86. The edges of proximal sheath 85 and distal sheath 86 meet or overlap near the middle, for example, of balloon 84 so that therapeutic agent 18 on balloon 84 is enclosed within the sheaths. Of course, the edges of proximal sheath 85 and distal sheath 86 can meet or overlap at another portion of balloon 84. In addition, as an alternate embodiment, the edges of the proximal sheath 85 and the distal sheath 86 may be separated, in which case it may be desirable to have the therapeutic agent only on those portions of the balloon covered by the sheaths.

In operation, balloon 84 is inserted into the body via a catheter, with the balloon in an unexpanded condition as shown in FIG. 4A. Because therapeutic agent 18 is covered by sheaths 85 and 86, therapeutic agent 18 is protected while balloon 84 is being guided to the target site. At the target site, as shown in FIG. 4B, balloon 84 is inflated. Expansion of balloon 84 causes sheaths 85 and 86 to slip across the surface of balloon 84 and be pulled proximally and distally, respectively, thus exposing therapeutic agent 18 for release at the target site. In an alternate embodiment, therapeutic agent 18 is applied to the inner surface of sheaths 85 and 86 so that therapeutic agent 18 is smeared onto the surface of balloon 84 as the sheaths retract.

In certain embodiments, the sheath comprises a body and a plurality of reservoirs for containing a therapeutic agent. The reservoirs can be, for example, longitudinally-extending channels within the body of the sheath. As the balloon is expanded within the sheath, the expanding balloon applies pressure against the reservoirs to push the therapeutic agent out of the reservoirs. In some cases, two or more of the reservoirs may be in communication with each other.

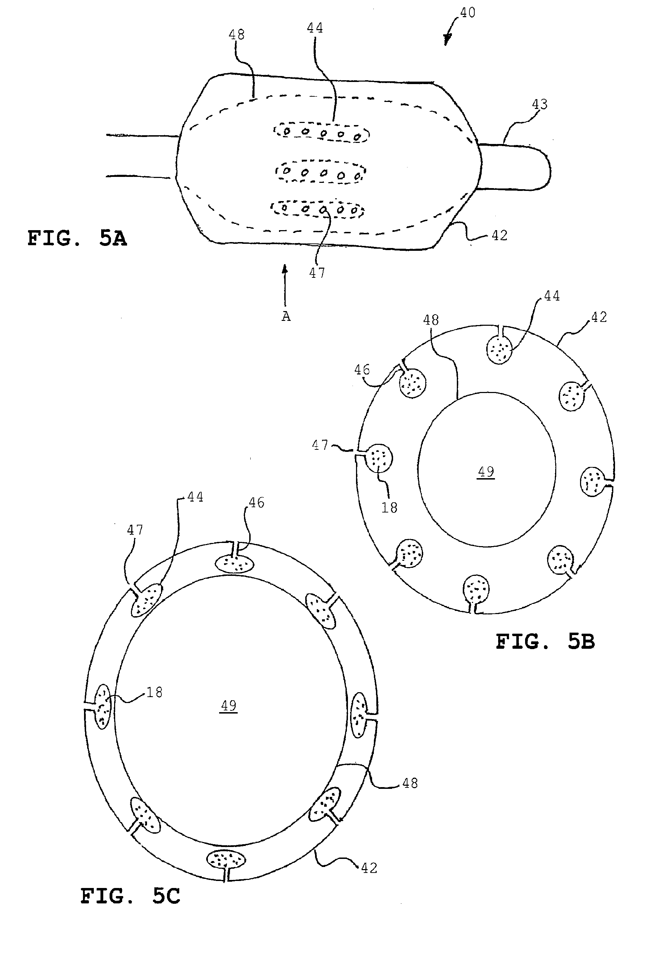

For example, referring to the embodiment shown in FIGS. 5A-5C, a catheter device 40 comprises a balloon 48 mounted on an elongate shaft 43 (note that balloon 48 may have a smaller uninflated profile relative to shaft 43 than what is depicted in FIG. 5A). Balloon 48 is enclosed in a sheath 42 that is shaped and dimensioned to allow balloon 48 to expand within sheath 42. Sheath 42 has a plurality of longitudinally extending channels 44 which contain a therapeutic agent 18. Each channel 44 is in communication with a plurality of pores 47 on the external surface of sheath 42 via openings 46. As shown in FIG. 5B, when balloon 48 (shown with inflation chamber 49) is in a deflated state, channels 44 are in a relaxed condition. FIG. 5B shows a space between channels 44 and balloon 48 which is for illustration purposes only. This space is not necessary and channels 44 may be in contact with un-inflated balloon 48. The sheath 42 may be fitted snugly around the un-inflated balloon 48.

In operation, catheter device 40 is inserted into the body via a catheter. At the target site, as shown in FIG. 5C, balloon 48 is inflated, causing it to press against channels 44. Internal pressure in channels 44 causes therapeutic agent 18 to be forced through openings 46 and out of pores 47 of sheath 42.

In certain embodiments, the sheath has a non-linear compliance curve. Balloons having high compliance at smaller inflation diameters and low compliance at larger inflation diameters (i.e., a non-linear or hybrid compliance curve) are described in U.S. Patent No. 5,348,538 (Wang et al.), which is incorporated by reference herein. Such balloons can be made using the shrunken balloon technique described in Wang et al., which is incorporated by reference herein. In general, a high compliance balloon will undergo a relatively large increase in diameter in response to an increase in inflation pressure, whereas a low compliance balloon will undergo a relatively small increase in diameter in response to an increase in inflation pressure. As such, a sheath of the present invention can be made in the same or similar manner, such that it has high compliance at smaller expansion diameters and low compliance at larger expansion diameters. In some cases, such a sheath can be formed of ePTFE having a porous cell structure. As the sheath expands in diameter with inflation of the balloon, the size of the pores in the sheath increase, making it more permeable to the therapeutic agent.

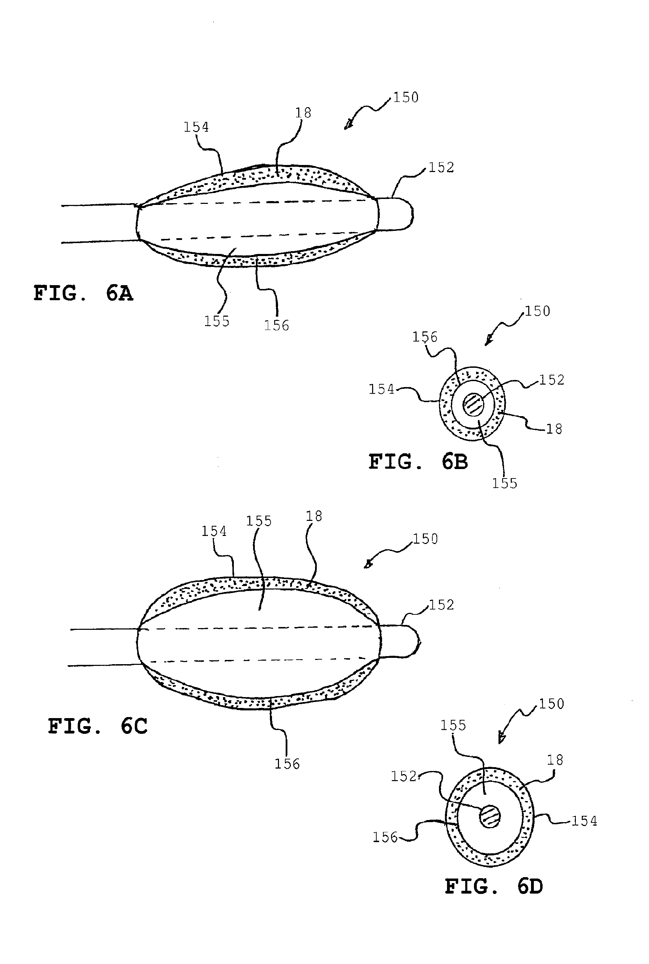

For example, referring to the embodiment shown in FIGS. 6A-6D, a catheter device 150 comprises a balloon 156 mounted on an elongate shaft 152. Balloon 156 has an inflation chamber 155. Balloon 156 is covered with a sheath 154 formed of ePTFE having a porous cell structure. Sheath 154 has a non-linear compliance curve, such that it has high compliance at smaller expansion diameters and low compliance at larger expansion diameters. Disposed between the outer surface of balloon 156 and sheath 154 is a therapeutic agent 18.

In operation, balloon 156 is inserted into the body via a catheter. Because therapeutic agent 18 is covered by sheath 154, therapeutic agent 18 is protected while balloon 156 is being guided to the target site. At the target site, as shown in FIGS. 6C and 6D, balloon 156 is inflated. With the inflation of balloon 156, sheath 154 also expands. Initially, sheath 154 expands with relatively high compliance. Also, as sheath 154 expands, the pores in sheath 154 increase in size, allowing the release of therapeutic agent 18 through sheath 154. With further inflation of balloon 156 and expansion of sheath 154, sheath 154 becomes less compliant as it reaches its predetermined maximum diameter.

In certain embodiments, it may be desirable to apply the sheath (e.g., of ePTFE) over a balloon that has been inflated and then deflated. This deflation or shrinking process can allow subsequent over-expansion of the sheath or ePTFE layer to create a larger cell size to allow for burst release of the therapeutic agent. When a relatively non-compliant balloon is used, the shrinking process facilitates the use of higher inflation pressures.

In certain embodiments, the sheath may be elastomeric and expands upon inflation of the balloon. In some cases, the elastomeric sheath may be an elastomeric mesh. With expansion, the widened spaces of the mesh allow for the release of the therapeutic agent. In some cases, the elastomeric mesh may comprise a biodegradable material (e.g., polyglycolic acid, polylactic acid, polyanhydride, etc.). In some cases, the medical device may further include a biodegradable stent (e.g., made of a biodegradable polymer or bioresorbable metal, such as magnesium) disposed over the balloon, with the biodegradable stent being covered by the elastomeric mesh.

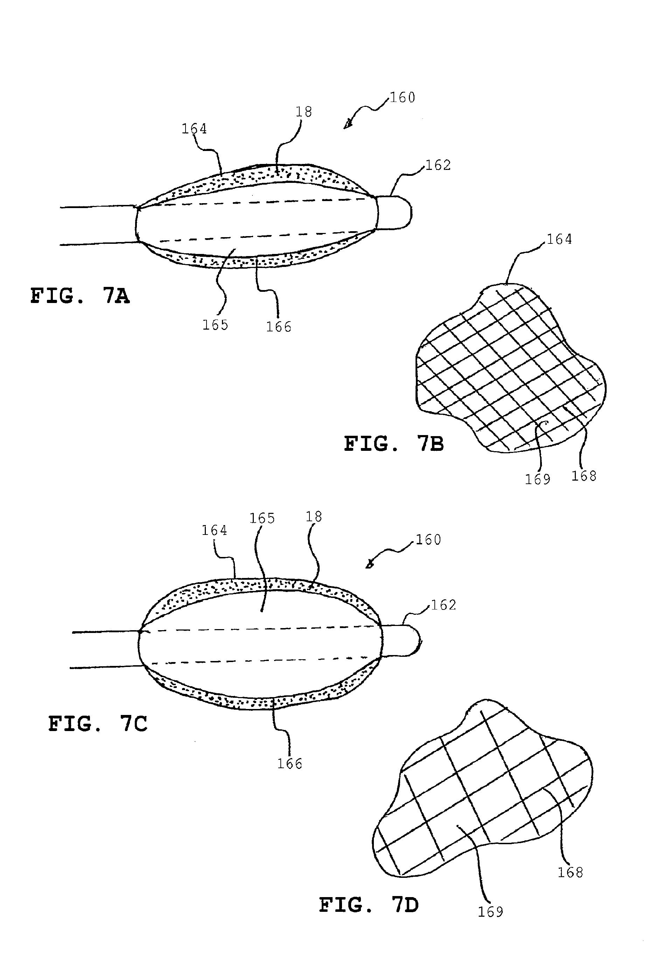

For example, referring to the embodiment shown in FIGS. 7A-7D, a catheter device 160 comprises a balloon 166 mounted on an elongate shaft 162. Balloon 166 has an inflation chamber 165. Balloon 166 is covered with an expandable sheath 164 formed of an elastomeric mesh 168 having interstices 169 (see FIG. 7B). Disposed between the outer surface of balloon 166 and sheath 164 is a therapeutic agent 18. With balloon 166 in a deflated state and sheath 154 unexpanded, intersticial openings 169 are sufficiently small such that therapeutic agent 18 is trapped by elastomeric mesh 168 of sheath 164 (e.g., less than 25 µm, or less than 10 µm in size, or another suitable size depending upon the particular application).

In operation, balloon 166 is inserted into the body via a catheter. Because therapeutic agent 18 is covered by sheath 164, therapeutic agent 18 is protected while balloon 166 is being guided to the target site. At the target site, as shown in FIG. 7C, balloon 166 is inflated. With the inflation of balloon 166, sheath 164 also expands. Expansion of sheath 164 causes the widening of intersticial openings 169 (see FIG. 7D), which allows for the release of therapeutic agent 18.

In some cases, sheath 164 may further have numerous pili as shown in FIG. 3. In such cases, therapeutic agent 18 that passes through the wall of sheath 164 may then be "smeared" onto the inner wall of the blood vessel when the balloon is retracted through the blood vessel. In such cases, the therapeutic agent may be formulated with a biocompatible adhesive substance.

In certain embodiments, the sheath is designed to tear, break, or otherwise become disrupted at one or more points when the sheath expands as a result of balloon inflation. The sheath is provided with one or more weakened portions where the tearing, breaking, or disruption occurs. The weakened portions are areas where the sheath has a different structure or composition than other areas of the sheath, wherein the different structure or composition at the weakened portions cause the area to be structurally weaker than the other areas of the sheath. For example, the weakened portions may be slots, slits, grooves, or perforations in the sheath. The weakened portions may have any of various orientations or configurations relative to the balloon, including radial, longitudinal, grid-like, or random. The tearing, breaking, or disruption of the sheath allows the release of the therapeutic agent. In some cases, the weakened portions may be areas where the sheath is attached to the balloon such that disruption of the sheath preferentially occurs at the attachment points. Such attachments can be made by, for example, spot welding of the sheath to the balloon.

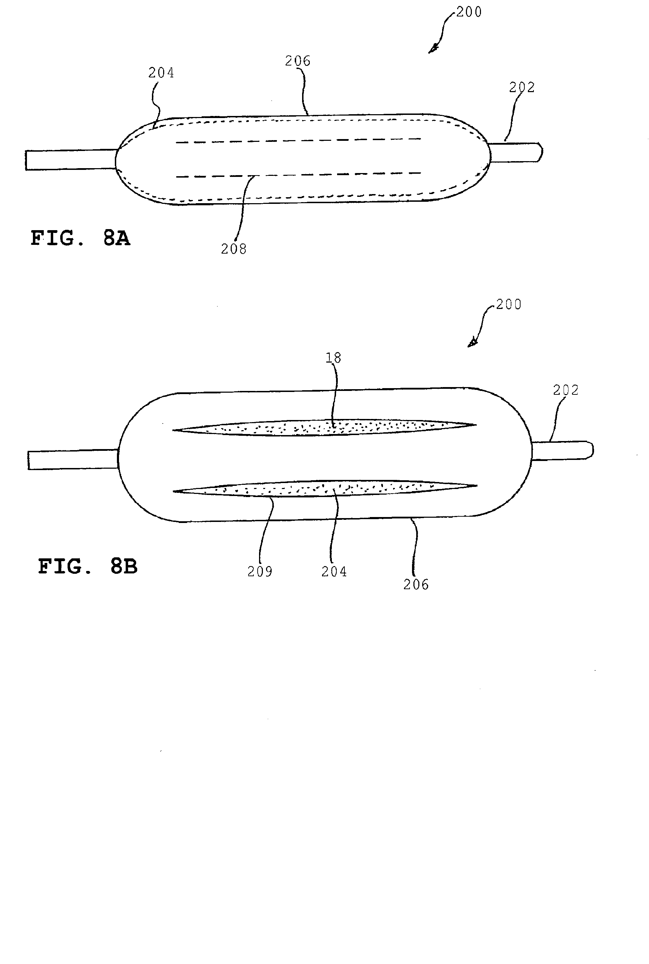

Referring to the embodiment shown in FIGS. 8A and 8B, a catheter device 200 comprises a balloon 204 mounted on an elongate shaft 202. Balloon 204 is coated with a therapeutic agent 18 (not shown in FIG. 8A). Balloon 204 is enclosed within a sheath 206 such that therapeutic agent 18 is covered by sheath 206. Sheath 206 has a plurality of longitudinally extending perforation lines 208, along which sheath 206 is designed to tear.

In operation, balloon 204 (enclosed within sheath 206) is inserted into the body via a catheter. Because therapeutic agent 18 is covered by sheath 206, therapeutic agent 18 is protected while balloon 204 is being guided to the target site. At the target site, as shown in FIG. 8B, balloon 204 is inflated, causing sheath 206 to also expand. Expansion of sheath 206 causes tearing of sheath 206 along perforation lines 208 to form tears 209. Tears 209 expose therapeutic agent 18 on the surface of balloon 204 such that therapeutic agent 18 is released.

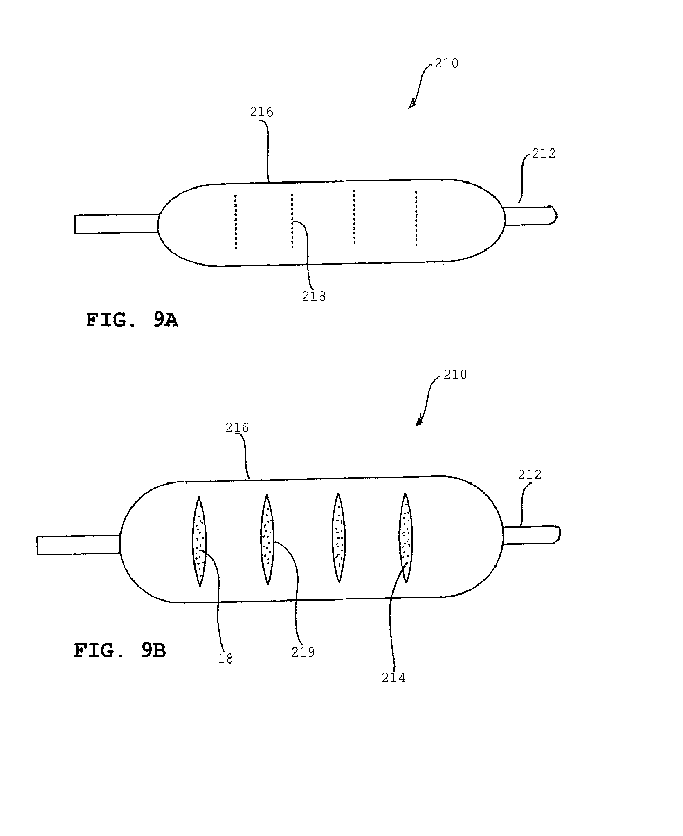

Referring to the embodiment shown in FIGS. 9A and 9B, a catheter device 210 comprises a balloon 214 (not shown in FIG. 9A) mounted on an elongate shaft 212. Balloon 214 is coated with a therapeutic agent 18 (not shown in FIG. 9A). Balloon 214 is enclosed within a sheath 216 such that therapeutic agent 18 is covered by sheath 216. Sheath 216 has a plurality of radially extending perforation lines 218, along which sheath 216 is designed to tear.

In operation, balloon 214 enclosed within sheath 216 is inserted into the body via a catheter. Because therapeutic agent 18 is covered by sheath 216, therapeutic agent 18 is protected while balloon 214 is being guided to the target site. At the target site, as shown in FIG. 9B, balloon 214 is inflated, causing sheath 216 to also expand. Expansion of sheath 216 causes tearing of sheath 216 along perforation lines 218 to form tears 219. Tears 219 expose therapeutic agent 18 on the surface of balloon 214 such that therapeutic agent 18 is released.

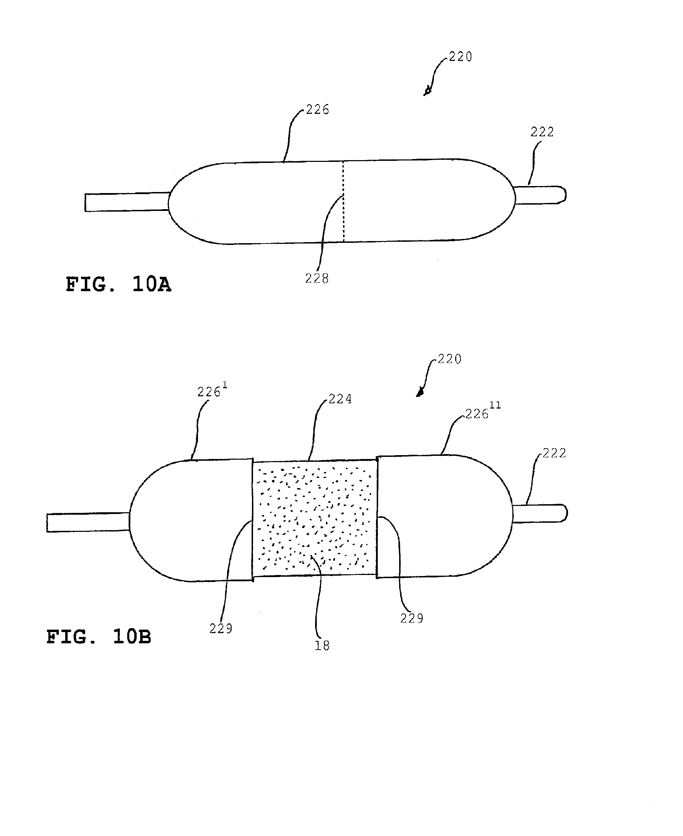

Referring to the embodiment shown in FIGS. 10A and 10B, a catheter device 220 comprises a balloon 224 (not shown in FIG. 10A) mounted on an elongate shaft 222. Balloon 224 is coated with a therapeutic agent 18 (not shown in FIG. 10A). Balloon 224 is enclosed within a sheath 226 such that therapeutic agent 18 is covered by sheath 226. At its midportion, sheath 226 has a circumferentially extending perforation line 228, along which sheath 226 is designed to tear.

In operation, balloon 224 enclosed within sheath 226 is inserted into the body via a catheter. Because therapeutic agent 18 is covered by sheath 226, therapeutic agent 18 is protected while balloon 224 is being guided to the target site. At the target site, as shown in FIG. 10B, balloon 224 is inflated, causing sheath 226 to also expand. Expansion of sheath 226 causes tearing of sheath 226 at perforation line 228 (creating sheath edges 229) as proximal portion 226' and distal portion 226" of sheath 226 are pulled in opposite directions. This tearing of sheath 226 exposes therapeutic agent 18 on the surface of balloon 224 such that therapeutic agent 18 is released.

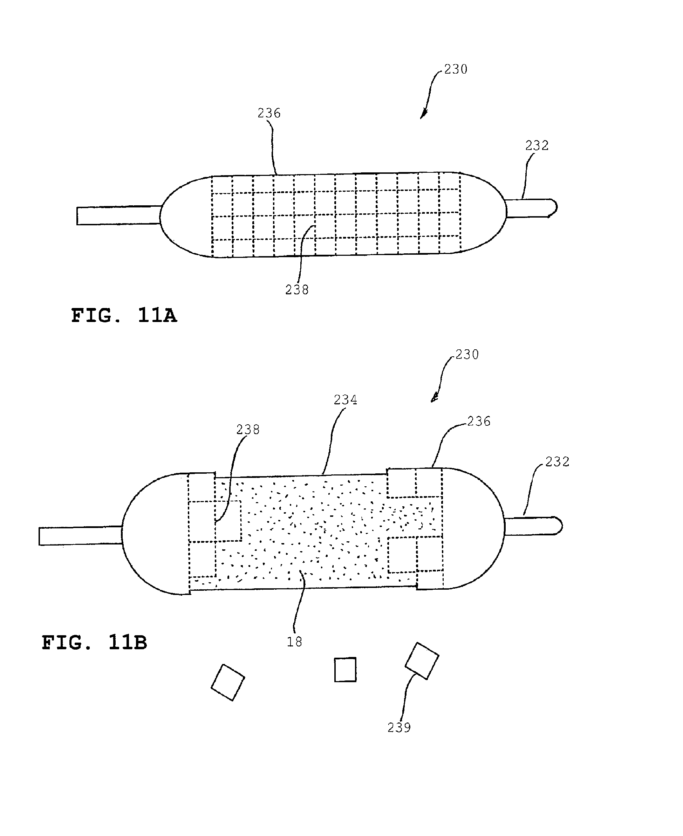

Referring to the embodiment shown in FIGS. 11A and 11B, a catheter device 230 comprises a balloon 234 (not shown in FIG. 11A) mounted on an elongate shaft 232. Balloon 234 is coated with a therapeutic agent 18 (not shown in FIG. 11A). Balloon 234 is enclosed within a sheath 236 such that therapeutic agent 18 is covered by sheath 236. Sheath 236 has a plurality of perforation lines 238 oriented in a grid-like pattern, along which sheath 236 is designed to tear. The size of at least some of the individual grid units may be in the micron range (e.g., less than 100 µm, or less than 10 µm) such that the fragments would not cause an embolism.

In operation, balloon 234 enclosed within sheath 236 is inserted into the body via a catheter. Because therapeutic agent 18 is covered by sheath 236, therapeutic agent 18 is protected while balloon 234 is being guided to the target site. At the target site, as shown in FIG. 11B, balloon 234 is inflated, causing sheath 236 to also expand. Expansion of sheath 236 causes tearing of sheath 236 at perforation lines 238, which results in the fragmentation of sheath 236 into fragments 239. The size of fragments 239 may be in the micron range (e.g., less than 100 µm, or less than 10 µm). This fragmentation of sheath 236 exposes therapeutic agent 18 on the surface of the balloon 234 such that therapeutic agent 18 is released.

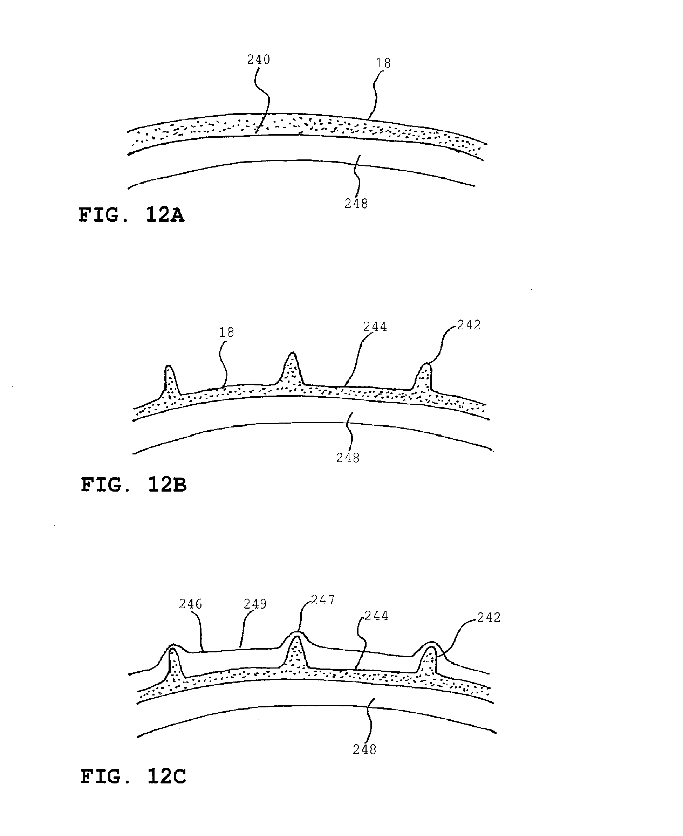

Referring to the embodiments shown in FIGS. 12A-12C, a catheter device comprises a balloon having a surface 240. As shown in FIG. 12A, a layer of therapeutic agent 18 is deposited onto surface 240 of the balloon. As shown in FIG. 12B, the layer of therapeutic agent 18 is then embossed with a pattern (e.g., by stamping) to create ridges 242 and depressions 244 in the layer of therapeutic agent 18. The layer of therapeutic agent 18 can be formulated in any suitable way to allow this type of patterning (e.g., by including a binder material, such as polyvinylpyrrolidone).

As shown in FIG. 12C, a sheath is provided by depositing a film 246 (representing a sheath) of brittle material, such as low molecular weight poly(lactic-co-glycolic acid) (PLGA) or low molecular weight poly(lactic acid) (PLA), onto the patterned layer of therapeutic agent 18. Film 246 may also be made of a non-polymeric material, such as salts of moderate to high molecular weight. Film 246 that is located over ridges 242 is thinner (at regions 247) relative to film 246 that is located over depressions 244 (at regions 249). Thus, a pattern of thin regions 247 in film 246 allows film 246 to fragment upon expansion of the balloon. In some cases, thin regions 247 may be attached to the surface 240 of the balloon (e.g., by spot welding), which can enhance fragmentation of film 246.

In certain embodiments, the sheath comprises nanofibers. The nanofibers are formed of any suitable polymer material, including polymers that are elastic. The nanofibers may have diameters (thickness) that are less than 1 µm (e.g., in the range of 200 nm - 1 µm). The nanofibers may comprise biodegradable polymers, such as poly(lactic-co-glycolic acid) (PLGA), poly(lactic acid) (PLA), or uncured poly(ethylene oxide) (PEO). The nanofibers may also comprise non-biodegradable polymers, such as poly(styrene-isobutylene-styrene) (SIBS) block copolymers, polyamides (e.g., nylon), polyesters, polyethylene, polyurethane, or carbon fiber.

The nanofibers may be disposed over the balloon in any of various ways. In some cases, the nanofibers are wound circumferentially around the body of the balloon. The nanofibers may be wound around the balloon loosely or under elastic tension. Where the nanofibers are wound around the balloon under elastic tension, the elastic tension may restrain the balloon in its unexpanded or folded configuration.

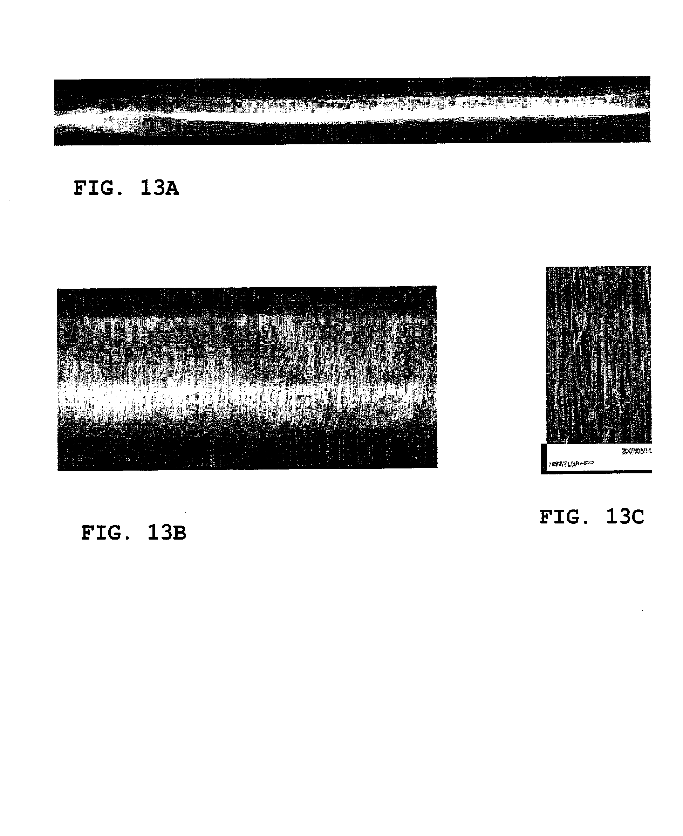

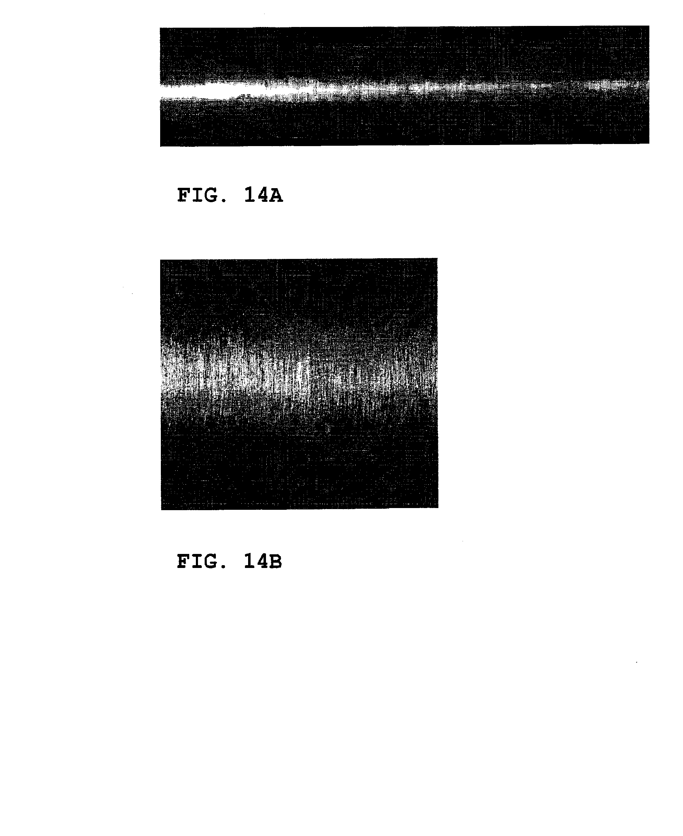

For example, FIGS. 13A-13C show PLGA nanofibers wrapped circumferentially around a balloon in its folded configuration. FIG. 13A shows a side view of the balloon; FIG. 13B shows a close-up view of a portion of the balloon, with the nanofibers being more visible; and FIG. 13C shows a scanning-electron micrograph of the nanofiber sheath showing the individual nanofibers. In another example, FIGS. 14A and 14B show PLGA nanofibers wrapped circumferentially around a balloon in its inflated configuration. FIG. 14A shows a side view of the balloon; and FIG. 14B shows a close-up view of a portion of the balloon, with the nanofibers being more visible.

In some cases, the nanofibers are deposited as a tangled mat on the balloon. In some of such cases, additional nanofibers may be wound circumferentially around the tangled mat of nanofibers (e.g., to encase the tangled mat of nanofibers).

The nanofibers may be disposed over the balloon with the balloon in any suitable configuration (e.g., inflated or uninflated, folded or unfolded). The nanofibers may be disposed over the entire balloon or over only a portion of the balloon (e.g., between the cones).

The therapeutic agent may be associated with the nanofiber sheath in any of various ways. The therapeutic agent may be provided in any suitable form, including as a liquid, a gel, or a solid. In some cases, the therapeutic agent is contained between the nanofiber sheath and the balloon (i.e., the sheath covers over the therapeutic agent). For example, the therapeutic agent can be deposited on the balloon and the nanofibers are spun (e.g., by electro-spinning) over the therapeutic agent to encase and protect it. Where the sheath comprises a tangled mat of nanofibers, the therapeutic agent may be dispersed within the network of nanofibers. In some cases, the therapeutic agent may be dispersed in the spaces between the nanofibers. In some cases, where the nanofibers are biodegradable (e.g., made of partially cured PEO), the therapeutic agent may constitute part of the composition of the nanofibers and be released upon degradation of the nanofibers. For example, the therapeutic agent may be contained in the nanofiber solution and spun with the nanofibers.

Upon delivery of the balloon to the target site, the therapeutic agent may be released through any of various mechanisms. Where the nanofiber sheath covers over the therapeutic agent, the therapeutic agent may be released through gaps or openings between the nanofibers. For example, as the balloon expands, the therapeutic agent may be squeezed through such gaps or openings. In some cases, stretching of the nanofibers as the balloon expands causes thinning of the nanofibers. This thinning of the nanofibers increases the spacing between the nanofibers, providing openings for the release of the therapeutic agent.

In addition to their role in delivery of the therapeutic agent, the nanofibers may also serve other functions. For example, elastic nanofibers may serve to control the folding or refolding of the balloon or control the shape or dimensions of the expanded balloon.

In another aspect of the present invention, the balloon includes a micro-electromechanical system (MEMS) for drug delivery. Various types of drug delivery systems using MEMS are known in the art, such as microfluidic devices that incorporate micropumps, valves, or flow channels; microfabricated porous membranes for drug encapsulation; microparticles for carrying drugs; and the microchip devices for drug delivery described in U.S. Patent No. 6,656,162 (Santini et al.).

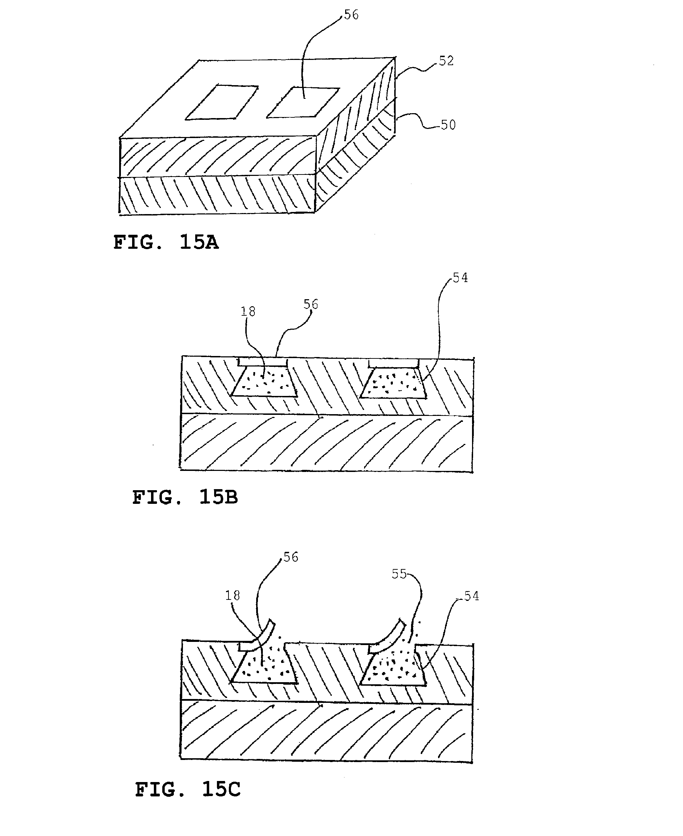

Referring to FIGS. 15A-15C, in this embodment, a catheter device comprises a balloon having a substrate layer 52 on the surface of the balloon wall 50. Substrate layer 52 has a plurality of micro-sized reservoirs 54 which contain a therapeutic agent 18. Reservoirs 54 are formed using any of various microfabrication techniques, including lithographic etching, molding, or micromachining (e.g., laser drilling). As such, substrate layer 52 is made of a material which can be shaped by microfabrication techniques, including for example, ceramics, metal oxides, semiconductor materials, and polymers. Reservoirs 54 have openings 55 through which therapeutic agent 18 may be released. To contain therapeutic agent 18 within the reservoirs 54 until the appropriate time for release, reservoirs 54 are covered by reservoir caps 56 formed of a shape memory material. Various types of shape memory material are suitable for use in reservoir caps 56, including shape memory polymers and shape memory alloys (e.g., nitinol).

In operation, the balloon is inserted into the body via a catheter. At the target site, as shown in FIG. 15C, the balloon is inflated and reservoir caps 56 are actuated to open (e.g., by bending), allowing therapeutic agent 18 contained in reservoirs 54 to be released. Reservoir caps 56 may be actuated in any of various ways, including temperature change or application of an electric current.

In certain embodiments of the invention, as described above, the configuration of the balloon and the sheath can be controlled such as to allow release of the therapeutic agent only at the desired time. For example, the device may be designed such that a certain pressure within the balloon is required for the sheath to release the therapeutic agent (e.g., by retracting, fracturing, fragmenting, opening pores, etc.). In this way, the therapeutic agent can be held in the folds while the device is delivered through the blood vessel to the target site. Then, at the target site, the balloon is inflated to the pressure and/or diameter at which the sheaths are designed to release the therapeutic agent. In this way, for example, the therapeutic agent release can be controlled such that it is released only if the balloon is in contact with or in close proximity to the vessel wall. This helps to prevent loss of the therapeutic agent during catheter placement and balloon inflation. Also, because deflation of the balloon can, in some instances, stop or substantially reduce therapeutic agent release, certain embodiments of the invention can control the duration of release after the initial release of therapeutic agent.

Medical devices of the present invention may also include a vascular stent mounted on the balloon. The vascular stent may be any of those known in the art, including those with or without coatings that elute a therapeutic agent. The stent may also be biostable, bioerodable, or biodegradable.

The balloons of the present invention may also be coated with a low-molecular weight carbohydrate, such as mannitol. The carbohydrate may be a separate coating or be blended with the therapeutic agent. The balloons of the present invention may also be coated with a radiocontrast agent (ionic or non-ionic), such as iopromide. The contrast agent may be a separate coating or be blended with the therapeutic agent.

The therapeutic agent used in the present invention may be any pharmaceutically-acceptable agent such as a drug, a non-genetic therapeutic agent, a biomolecule, a small molecule, or cells. Example drugs include anti-proliferative agents or anti-restenosis agents such as paclitaxel, sirolimus (rapamycin), tacrolimus, everolimus, and zotarolimus.

Exemplary non-genetic therapeutic agents include anti-thrombogenic agents such heparin, heparin derivatives, prostaglandin (including micellar prostaglandin E1), urokinase, and PPack (dextrophenylalanine proline arginine chloromethylketone); anti-proliferative agents such as enoxaparin, angiopeptin, sirolimus (rapamycin), tacrolimus, everolimus, zotarolimus, monoclonal antibodies capable of blocking smooth muscle cell proliferation, hirudin, and acetylsalicylic acid; anti-inflammatory agents such as dexamethasone, rosiglitazone, prednisolone, corticosterone, budesonide, estrogen, estrodiol, sulfasalazine, acetylsalicylic acid, mycophenolic acid, and mesalamine; anti-neoplastic/anti-proliferative/anti-mitotic agents such as paclitaxel, epothilone, cladribine, 5-fluorouracil, methotrexate, doxorubicin, daunorubicin, cyclosporine, cisplatin, vinblastine, vincristine, epothilones, endostatin, trapidil, halofuginone, and angiostatin; anti-cancer agents such as antisense inhibitors of c-myc oncogene; antimicrobial agents such as triclosan, cephalosporins, aminoglycosides, nitrofurantoin, silver ions, compounds, or salts; biofilm synthesis inhibitors such as non-steroidal anti-inflammatory agents and chelating agents such as ethylenediaminetetraacetic acid, O,O'-bis (2-aminoethyl) ethyleneglycol-N,N,N',N'-tetraacetic acid and mixtures thereof; antibiotics such as gentamycin, rifampin, minocyclin, and ciprofloxacin; antibodies including chimeric antibodies and antibody fragments; anesthetic agents such as lidocaine, bupivacaine, and ropivacaine; nitric oxide; nitric oxide (NO) donors such as linsidomine, molsidomine, L-arginine, NO-carbohydrate adducts, polymeric or oligomeric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, warfarin sodium, Dicumarol, aspirin, prostaglandin inhibitors, platelet aggregation inhibitors such as cilostazol and tick antiplatelet factors; vascular cell growth promotors such as growth factors, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vascoactive mechanisms; inhibitors of heat shock proteins such as geldanamycin; angiotensin converting enzyme (ACE) inhibitors; beta-blockers; βAR kinase (βARK) inhibitors; phospholamban inhibitors; protein-bound particle drugs such as ABRAXANE™; structural protein (e.g., collagen) cross-link breakers such as alagebrium (ALT-711); any combinations and prodrugs of the above.

Exemplary biomolecules include peptides, polypeptides and proteins; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Nucleic acids may be incorporated into delivery systems such as, for example, vectors (including viral vectors), plasmids or liposomes.

Non-limiting examples of proteins include serca-2 protein, monocyte chemoattractant proteins (MCP-1) and bone morphogenic proteins ("BMP's"), such as, for example, BMP-2, BMP-3, BMP-4, BMP-5, BMP-6 (VGR-1), BMP-7 (OP-1), BMP-8, BMP-9, BMP-10, BMP-11, BMP-12, BMP-13, BMP-14, BMP-15. Preferred BMP's are any of BMP-2, BMP-3, BMP-4, BMP-5, BMP-6, and BMP-7. These BMPs can be provided as homodimers, heterodimers, or combinations thereof, alone or together with other molecules. Alternatively, or in addition, molecules capable of inducing an upstream or downstream effect of a BMP can be provided. Such molecules include any of the "hedghog" proteins, or the DNA's encoding them. Non-limiting examples of genes include survival genes that protect against cell death, such as anti-apoptotic Bcl-2 family factors and Akt kinase; serca 2 gene; and combinations thereof. Non-limiting examples of angiogenic factors include acidic and basic fibroblast growth factors, vascular endothelial growth factor, epidermal growth factor, transforming growth factors α and β, platelet-derived endothelial growth factor, platelet-derived growth factor, tumor necrosis factor α, hepatocyte growth factor, and insulin-like growth factor. A non-limiting example of a cell cycle inhibitor is a cathespin D (CD) inhibitor. Non-limiting examples of anti-restenosis agents include p15, p16, p18, p19, p21, p27, p53, p57, Rb, nFkB and E2F decoys, thymidine kinase and combinations thereof and other agents useful for interfering with cell proliferation.

Exemplary small molecules include hormones, nucleotides, amino acids, sugars, and lipids and compounds have a molecular weight of less than 100kD.

Exemplary cells include stem cells, progenitor cells, endothelial cells, adult cardiomyocytes, and smooth muscle cells. Cells can be of human origin (autologous or allogenic) or from an animal source (xenogenic), or genetically engineered. Non-limiting examples of cells include side population (SP) cells, lineage negative (Lin⁻) cells including Lin⁻ CD34⁻, Lin⁻CD34⁺, Lin⁻cKit ⁺, mesenchymal stem cells including mesenchymal stem cells with 5-aza, cord blood cells, cardiac or other tissue derived stem cells, whole bone marrow, bone marrow mononuclear cells, endothelial progenitor cells, skeletal myoblasts or satellite cells, muscle derived cells, go cells, endothelial cells, adult cardiomyocytes, fibroblasts, smooth muscle cells, adult cardiac fibroblasts + 5-aza, genetically modified cells, tissue engineered grafts, MyoD scar fibroblasts, pacing cells, embryonic stem cell clones, embryonic stem cells, fetal or neonatal cells, immunologically masked cells, and teratoma derived cells. Any of the therapeutic agents may be combined to the extent such combination is biologically compatible.

The foregoing description and examples have been set forth merely to illustrate the invention and are not intended to be limiting. Each of the disclosed aspects and embodiments of the present invention may be considered individually or in combination with other aspects, embodiments, and variations of the invention. Modifications of the disclosed embodiments incorporating the spirit and substance of the invention may occur to persons skilled in the art and such modifications are within the scope of the present invention.

## Claims

1. A medical device comprising:
a catheter;
a balloon mounted on the catheter;
a sheath disposed around the balloon, wherein the sheath comprises nanofibers; and
a therapeutic agent retained on the medical device by the sheath.

2. The medical device of claim 1, wherein the therapeutic agent is disposed between the surface of the balloon and the sheath.

3. The medical device of claim 2, wherein the nanofibers are elastic, and wherein stretching of the nanofibers upon balloon expansion enlarges the spacing between the nanofibers.

4. The medical device of claim 2, wherein the therapeutic agent is squeezed through spaces between the nanofibers when the balloon is inflated.

5. The medical device of claim 1, wherein the nanofibers comprise a biodegradable polymer.

6. The medical device of claim 1, wherein the therapeutic agent is dispersed in the space between the nanofibers.
